(19) **Europäisches Patentamt**
**European Patent Office**
**Office européen des brevets**

(11) **EP 3 187 187 A1**

(12) **EUROPEAN PATENT APPLICATION**
published in accordance with Art. 153(4) EPC

(43) Date of publication:
**05.07.2017 Bulletin 2017/27**

(51) Int Cl.:
*A61K 33/44* [(2006.01)]     *A61P 1/16* [(2006.01)]
*A61P 13/12* [(2006.01)]     *A61P 39/02* [(2006.01)]

(21) Application number: **15836868.8**

(22) Date of filing: **27.08.2015**

(86) International application number:
**PCT/JP2015/074213**

(87) International publication number:
**WO 2016/031908 (03.03.2016 Gazette 2016/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **27.08.2014 JP 2014173157**

(71) Applicant: **Kureha Corporation**
**Chuo-ku**
**Tokyo 103-8552 (JP)**

(72) Inventors:
• **HONDA, Kaoru**
**Tokyo 103-8552 (JP)**
• **HIGASHIYAMA, Yukihiro**
**Tokyo 103-8552 (JP)**
• **WAKAHOI, Takashi**
**Tokyo 103-8552 (JP)**

(74) Representative: **Hoefer & Partner Patentanwälte mbB**
**Pilgersheimer Straße 20**
**81543 München (DE)**

(54) **ADSORBENT FOR ORAL ADMINISTRATION, THERAPEUTIC AGENT FOR RENAL DISEASES AND THERAPEUTIC AGENT FOR HEPATIC DISEASES**

(57)     An object of the present invention is to provide an adsorbent for oral administration capable of adsorbing large quantities of indole in the presence of bile acid.

    The above problem can be solved by an adsorbent for oral administration comprising a spherical activated carbon, the activated carbon having a specific surface area determined by the BET method of 800 m$^2$/g or more, a bulk density of from 0.3 g/mL to 0.8 g/mL, a volume of pores having a diameter less than 3 nm of 0.3 mL/g or more, and a micropore/mesopore ratio (Vm) determined by Formula (1): Vm = Vmic/Vmet (1) wherein Vmic is a volume of pores having a diameter less than 3 nm, and Vmet is a volume of pores having a diameter from 3 nm to 50 nm; of 3.0 or more.

EP 3 187 187 A1

**Description**

TECHNICAL FIELD

The present invention relates to an adsorbent for oral administration. The present invention also relates to an agent for treating or preventing a renal disease and an agent for treating or preventing a liver disease that contain the aforementioned adsorbent for oral administration as an active ingredient. The adsorbent for oral administration according to the present invention has high adsorption ability for indole in the presence of a high concentration of bile acid, indole being a precursor for indoxyl sulfate which is a poisonous toxic substance (toxin) in the body. According to the present invention, indole can be efficiently adsorbed even in the presence of cholic acid, which is contained in large quantities in bile.

BACKGROUND ART

Accompanying organ functional impairment in patients deficient in renal function or liver function, poisonous toxic substances accumulate and are produced in the body such as in the blood, and cause uremia or encephalopathy such as impaired consciousness. Since the number of such patients has shown an increasing trend year by year, the development of therapeutic medicines or organ substitute devices that has the function of removing toxic substances to outside the body in place of these deficient organs is a critical topic. Hemodialysis using artificial kidneys to removal of poisonous substances is currently the method most widely used. However, such hemodialysis-type artificial kidneys are not necessarily satisfactory due to problems such as the necessity for a specialized technician from the viewpoint of safety management because a special machine is used, and additionally, the high physical, mental and economic burden placed on the patient due to extracorporeal removal of blood, and the like.

As a means for solving these problems, an oral adsorbent that can be orally ingested and can treat functional impairment of the kidney or liver has been developed and used (Patent Document 1). The oral adsorbent has been widely clinically used in, for example, patients with hepatorenal functional impairment as an oral agent for treatment that has less side effect such as constipation. The oral adsorbent contains a porous spherical carbonaceous substance (that is, a spherical activated carbon) having a certain functional group, is highly safe to the body and stable, has excellent adsorbance of poisonous substances (that is, $\beta$-aminoisobutyric acid, $\gamma$-amino-n-butyric acid, dimethylamine, and octopamine), and also has beneficial selective adsorbance in the sense that it adsorbs little of the beneficial components in the intestines such as digestive enzymes and the like. Furthermore, the adsorbent described in Patent Document 1 uses pitch such as petroleum pitch as a carbon source, and is produced by performing oxidation treatment and reduction treatment after preparation of a spherical activated carbon.

On the other hand, it is known that in chronic renal failure patients, serum indoxyl sulfate concentration sometimes increases to approximately 60 times that of normal people, and it is also known that serum indoxyl sulfate concentration is reduced and the progression of renal failure is slowed by administration of the oral adsorbent described in Patent Document 1 (Non-Patent Documents 1 and 2).

CITATION LIST

Patent Literature

Patent Document 1: Japanese Examined Patent Application Publication No. S62-11611B
Patent Document 2: Japanese Unexamined Patent Application Publication No. 2006-273772A
Patent Document 3: Japanese Unexamined Patent Application Publication No. 2007-45775A

Non-Patent Literature

Non-Patent Document 1: Japanese Journal of Nephrology Vol. XXXII, No. 6 (1990), 65-71
Non-Patent Document 2: Japanese Journal of Clinical Dialysis Vol. 14, No. 4 (1998), 433-438

SUMMARY OF INVENTION

Technical Problem

Adsorption of toxic substances is an extremely important characteristic of an oral adsorbent containing a spherical activated carbon, but it is particularly important that indole, a precursor of indoxyl sulfate, which is a toxic substance in chronic renal failure patients, is adsorbed and removed from the intestinal environment. Patent Documents 2 and 3 disclose that an activated carbon having a volume of pores having a diameter 1.0 nm or less of from 0.2 to 2.5 mL/g has high indole adsorption performance. However, a large amount of bile acid (15 mM) is present in the human intestines. When the present inventors studied the indole adsorption ability of a conventional spherical activated carbon in the presence of cholic acid, which a primary component of bile acid, they found that an adsorbent for oral administration containing a conventional spherical activated carbon has lower indole adsorption ability in the presence of cholic acid. Therefore, an object of the present invention is to provide an adsorbent for oral administration capable of adsorbing large quantities of indole in the presence of bile acid.

Solution to Problem

As a result of diligent research on adsorbents for oral administration capable of adsorbing indole in large quantities in the presence of bile acid, the present inventors unexpectedly discovered that a spherical activated carbon having a reduced volume of mesopores relative to volume of micropores exhibits excellent toxic substance adsorption ability even in the presence of bile acid. That is, the above spherical activated carbon discovered by the present inventors is capable of adsorbing large quantities of toxic substances (particularly indole, a precursor of indoxyl sulfate) even in the presence of a high concentration of bile acid, and enables reduced dosage.
The present invention is based on such knowledge.
Therefore, the present invention relates to:

[1] An adsorbent for oral administration comprising a spherical activated carbon, wherein the activated carbon has a specific surface area determined by the BET method of 800 $m^2$/g or more, a bulk density of from 0.3 g/mL to 0.8 g/mL, a volume of pores having a diameter less than 3 nm of 0.3 mL/g or more, and a micropore/mesopore ratio (Vm) determined by Formula (1):

$$Vm = Vmic/Vmet \ (1)$$

wherein Vmic is a volume of pores having a diameter less than 3 nm, and Vmet is a volume of pores having a diameter from 3 nm to 50 nm; of 3.0 or more;
[2] The adsorbent for oral administration according to [1], wherein an average particle diameter of the spherical activated carbon is from 50 $\mu$m to 200 $\mu$m;
[3] The adsorbent for oral administration according to [1] or [2], wherein the spherical activated carbon is prepared from a crosslinked vinyl resin as a carbon source;
[4] An agent for treating or preventing a renal disease comprising, the adsorbent for oral administration according to any one of [1] to [3] as an active ingredient; and
[5] An agent for treating or preventing a liver disease comprising, the adsorbent for oral administration according to any one of [1] to [3] as an active ingredient.

Advantageous Effects of Invention

The adsorbent for oral administration according to the present invention can adsorb poisonous toxic substances in the intestines because adsorption ability for toxic substances in the presence of bile acid is high due to the use of a spherical activated carbon having a bulk density of from 0.3 g/mL to 0.8 g/mL, a high specific surface area, and a low volume of mesopores having a diameter from 3 nm to 50 nm relative to micropores having a diameter less than 3 nm. Therefore, the adsorbent for oral administration according to the present invention is effective as an agent for treating or preventing a renal disease or as an agent for treating or preventing a liver disease. Additionally, the dosage can be reduced to a dosage less than the dosage of a conventional adsorbent for oral administration.
In particular, the adsorbent for oral administration according to the present invention does not inhibit adsorption of toxic substances such as indole because there is little adsorption of cholic acid to the spherical activated carbon due to the volume of mesopores being small. As a result, it can exhibit excellent adsorption ability of toxic substances such as indole even in the presence of bile acid.

Brief Description of the Drawings

FIG. 1 is a graph of indole adsorption ability in the presence of cholic acid of the adsorbents for oral administration obtained in Examples 2 and 4 and Comparative Example 2, measured over time.
FIG. 2 is a diagram schematically representing the difference in indole adsorption in the presence of cholic acid in the adsorbent for oral administration of the present invention and a conventional adsorbent for oral administration.

Description of Embodiments

[1] Adsorbent for oral administration

The adsorbent for oral administration of the present invention comprises a spherical activated carbon, the carbon having a specific surface area determined by the BET method of 800 $m^2$/g or more, a bulk density of from 0.3 g/mL to 0.8 g/mL, a volume of pores having a diameter less than 3 nm of 0.3 mL/g or more, and a micropore/mesopore ratio (Vm) determined by Formula (1):

$$Vm = Vmic/Vmet \ (1)$$

wherein Vmic is a volume of pores having a diameter less than 3 nm, and Vmet is a volume of pores having a diameter from 3 nm to 50 nm; of 3.0 or more.

Specific Surface Area

The specific surface area of the spherical activated carbon can be determined by the BET method or the Langmuir method. The spherical activated carbon used for the adsorbent for oral administration according to the present invention has a specific surface area determined by the BET method (sometimes abbreviated as "SSA" hereinafter) of 800 $m^2$/g or more. When a spherical activated carbon has SSA of less than 800 $m^2$/g, indole adsorption performance in the presence of bile acid decreases, which is undesirable. The upper limit of SSA is not particularly limited, but from the perspective of bulk density and strength, SSA is preferably 3000 $m^2$/g or less.

Bulk Density

The bulk density of the spherical activated carbon used in the present invention is from 0.3 g/mL to 0.8 g/mL.
The upper limit of bulk density is preferably 0.75 g/mL or less and more preferably 0.70 g/mL or less. The lower limit of bulk density is 0.30 g/mL or more, preferably 0.40 g/mL or more, more preferably 0.45 g/mL or more, even more preferably 0.48 g/mL or more, and most preferably 0.50 g/mL or more. In the above range of bulk density, indole adsorption ability particularly in the presence of bile acid is excellent.
Furthermore, this is because a spherical activated carbon having low bulk density has excellent adsorption capacity for toxic substances, but, on the other hand, as bulk density decreases, the yield of a spherical activated carbon becomes worse, resulting in a decrease in economical viability of the production of the activated carbon. Additionally, when bulk density is too low, the spherical activated carbon is readily crushed and does not maintain a spherical shape because the strength of the spherical activated carbon decreases. Furthermore, in this specification, bulk density $\rho_B$ is the value obtained by dividing the dry weight W (g) of the spherical activated carbon when the spherical activated carbon is packed in a container by the volume V (mL) of the packed spherical activated carbon, and can be obtained by the following calculation formula.

[Equation 1]

$$\rho_{B(g/mL)} = \frac{W_{(g)}}{V_{(mL)}}$$

The bulk density of the spherical activated carbon is a good index for indicating the degree of activation. Specifically, the lower the bulk density, the more activation has proceeded. In the manufacturing process of the spherical activated carbon, in steam activation to be described later, relatively small pores are formed in the early phase of activation, and the pore diameter increases as activation proceeds, resulting in bulk density decreasing.

Average Particle Diameter

In this specification, an average particle diameter means the particle diameter at a cumulative particle diameter percentage of 50% on a volume standard cumulative particle diameter distribution curve (Dv50).

The range of average particle diameter of the spherical activated carbon used for the adsorbent for oral administration according to the present invention is not particularly limited, but is preferably from 0.01 mm to 1 mm. When the average particle diameter of the spherical activated carbon is less than 0.01 mm, the exterior surface area of the spherical activated carbon increases and adsorption of beneficial substances such as digestive enzymes readily occurs, which is undesirable. When the average particle diameter exceeds 1 mm, the diffusion length of toxic substances into the spherical activated carbon increases and the adsorption rate decreases, which is undesirable. The average particle diameter is preferably from 0.02 mm to 0.8 mm. This is because the spherical activated carbon having an average particle diameter particularly from 50 μm to 200 μm has excellent initial adsorption capacity, and in the general residence period in the upper small intestine, can very rapidly adsorb poisonous toxic substances in the body. A more preferred range of average particle diameter is from 50 μm to 170 μm, and an even more preferred range is from 50 μm to 150 μm.

Volume of Pores

According to the International Union of Pure and Applied Chemistry (IUPAC), pores 2 nm or less are defined as micropores, those from 2 nm to 50 nm are defined as mesopores, and those 50 nm or more are defined as macropores.

In the present specification, for convenience, "micropores" means pores having a pore diameter of less than 3 nm. "Mesopores" means pores having a pore diameter from 3 nm to 50 nm.

The spherical activated carbon used as the adsorbent for oral administration according to the present invention has a relatively small volume of mesopores compared to the volume of micropores. As a result, its indole adsorption performance in the presence of bile acid is high.

Volume of Micropores

The volume of pores having a diameter of less than 3 nm of the spherical activated carbon is 0.30 mL/g or more, preferably 0.35 mL/g or more, and more preferably 0.40 mL/g or more. Due to the volume of pores having a diameter less than 3 nm being 0.30 mL/g or more, indole can be adsorbed in large quantities. The upper limit of the volume of pores having a diameter less than 3 nm is not limited as long as the micropore/mesopore ratio is 3.0 or more, but it is preferably 2.0 mL/g or less, and more preferably 1.5 mL/g or less.

The volume of micropores having a diameter less than 3 nm can be measured by the nitrogen adsorption method, and can be analyzed by the Saito-Foley method (called "SF method" hereinafter), the Horvath-Kawazoe method, the density functional theory method, and the like, but in the present invention, volume obtained by the SF method, which assumes that pore shape is cylindrical, is used.

Volume of Mesopores

The volume of pores having a diameter from 3 nm to 50 nm of the spherical activated carbon is not particularly limited, but is preferably 0.40 mL/g or less, more preferably 0.35 mL/g or less, and even more preferably 0.30 mL/g or less. Due to the volume of pores having a diameter from 3 nm to 50 nm of the spherical activated carbon being 0.40 mL/g or less, adsorption of bile acid to mesopores can be suppressed. It is thought that adsorption of bile acid to mesopores inhibits adsorption of indole to micropores. Therefore, adsorption of indole to micropores is promoted by suppressing adsorption of bile acid to mesopores. The lower limit of the volume of pores having a diameter from 3 nm to 50 nm is not limited as long as the micropore/mesopore ratio is 3.0 or more, but it is preferably 0.005 mL/g or more, and more preferably 0.001 mL/g or more.

Micropore/Mesopore Ratio

The spherical activated carbon used in the present invention has a ratio of volume of micropores relative to volume of mesopores (also called "micropore/mesopore ratio" hereinafter) of 3.0 or more. Specifically, the micropore/mesopore ratio (Vm) determined by Formula (1):

$$Vm = Vmic/Vmet \ (1)$$

wherein Vmic is the volume of pores having a diameter less than 3 nm, and Vmet is the volume of pores having a diameter from 3 nm to 50 nm; is 3.0 or more.

The micropore/mesopore ratio of the spherical activated carbon used in the adsorbent for oral administration of the present invention is preferably 3.3 or more, more preferably 3.5 or more, and even more preferably 4.0 or more. The spherical activated carbon having a micropore/mesopore ratio of 3.0 or more can exhibit excellent indole adsorption performance in the presence of cholic acid.

The upper limit of the micropore/mesopore ratio of the spherical activated carbon used in the present invention is not particularly limited, but is preferably 16 or less. This is because the spherical activated carbon having a micropore/mesopore ratio of greater than 16 often has a volume of micropores having a diameter less than 3 nm of less than 0.30 mL/g and its indole adsorption quantity is low. This is also because it often has SSA of less than 800 m$^2$/g and the adsorbed quantity of toxic substances other than indole may be low.

The spherical activated carbon used in the present invention is not particularly limited, but it may be a surface-modified spherical activated carbon having total acidic group content of 0.30 meq or more, or it may be a surface-unmodified spherical activated carbon having total acidic group content of less than 0.30 meq. Regardless of whether or not it has been surface-modified, the spherical activated carbon of the present invention can exhibit excellent adsorption ability of toxic substances such as indole even in the presence of bile acid.

A surface-unmodified spherical activated carbon is a porous body obtained by performing activation treatment after heat-treating a carbon precursor. It may be a spherical activated carbon which has not subsequently undergone surface modification treatment by oxidation treatment and reduction treatment after activation treatment, or it may be a spherical activated carbon obtained by performing heat treatment in a non-oxidative atmosphere after the aforementioned activation treatment. From the perspective of functional group configuration, a surface-unmodified spherical activated carbon means a spherical activated carbon having total acidic group content of less than 0.30 meq/g. The total acidic group content is preferably 0.25 meq/g or less, and more preferably 0.20 meq/g or less.

A surface-modified spherical activated carbon is a porous body obtained by performing activation treatment after heat-treating a carbon precursor, and then further performing surface modification treatment by oxidation treatment, or surface modification treatment by oxidation treatment and reduction treatment. It can exhibit appropriate degrees of interaction with acids and bases. From the perspective of functional group configuration, a surface-modified spherical activated carbon means a spherical activated carbon having acidic centers of 0.30 meq/g or more. In particular, a surface-modified spherical activated carbon having total acidic group content of from 0.30 meq/g to 1.20 meq/g and total basic group content of from 0.20 meq/g to 0.9 meq/g is preferred because adsorption performance for water-soluble toxins such as DL-$\beta$-aminoisobutyric acid is high. In particular, the total acidic group content is preferably from 0.30 meq/g to 1.00 meq/g, and the total basic group content is preferably from 0.30 meq/g to 0.70 meq/g.

Diameter

The diameter of the spherical activated carbon used for the adsorbent for oral administration according to the present invention is not particularly limited, but is preferably 0.01 mm to 1 mm, and more preferably from 0.02 mm to 0.8 mm. When the diameter of the spherical activated carbon is less than 0.01 mm, the exterior surface area of the spherical activated carbon increases and adsorption of beneficial substances such as digestive enzymes readily occurs, which is undesirable. When the diameter exceeds 1 mm, the diffusion length of toxic substances into the spherical activated carbon increases and the adsorption rate decreases, which is undesirable.

Carbon Source

The spherical activated carbon used for the adsorbent for oral administration of the present invention may use any carbon-containing material as a carbon source. Examples of carbon-containing materials that can be used include synthetic resin and pitch. As the synthetic resin, heat-fusible resin or heat-infusible resin may be used. Here, heat-fusible resin is resin that ends up melting or decomposing as the temperature rises when activation treatment is performed without infusibility treatment, and that cannot yield an activated carbon. However, when activation treatment is performed after infusibility treatment has been performed, the heat-fusible resin can be used as an activated carbon. In contrast, heat-infusible resin carbonizes without melting as the temperature rises even when activation treatment is performed without infusibility treatment, and can yield an activated carbon. Furthermore, infusibility treatment means, for example, oxidation treatment at a temperature from 150°C to 400°C in an atmosphere containing oxygen, as will be described later.

A typical example of heat-fusible resin is thermoplastic resin, for example, crosslinked vinyl resin. On the other hand, a typical example of heat-infusible resin is thermosetting resin, examples of which include phenol resin and furan resin. Among known thermoplastic resins and thermosetting resins, any that can form spheres may be used. Furthermore, the above-described infusibility treatment is required when obtaining a spherical activated carbon from crosslinked vinyl resin, whereas it is unnecessary when obtaining a spherical activated carbon from ion exchange resin produced by adding a functional group to crosslinked vinyl resin. This is because crosslinked vinyl resin is considered to have been modified from heat-fusible resin to heat-infusible resin by the introduced functional group or functional group addition treatment. That is, crosslinked vinyl resin is included among heat-fusible resins in this specification, whereas ion exchange resin is included among heat-infusible resins in this specification.

The carbon source of the spherical activated carbon used in the present invention is not particularly limited, but the use of a synthetic resin is preferred due to its ease of handling. Examples of synthetic resins include thermosetting resins which are heat-infusible resins (for example, phenol resin and furan resin) and ion exchange resins, which are heat-infusible resins, and thermoplastic resins (for example, crosslinked vinyl resin), which are heat-fusible resins. Here, with thermosetting resins, voids are readily formed in the spherical activated carbon and strength decreases, and when crushed, there is the danger of it piercing the intestines. With ion exchange resin, attention is required when used in oral administration because the ion exchange resin contains sulfur components, and the like. Accordingly, use of a thermoplastic resin (for example, crosslinked vinyl resin) as the carbon source of the spherical activated carbon is more preferred.

Operations

The reason that the adsorbent for oral administration of the present invention exhibits excellent indole adsorption ability even in the presence of cholic acid has not been completely elucidated, but can be theorized as follows. However, the present invention is not limited by the following explanation.

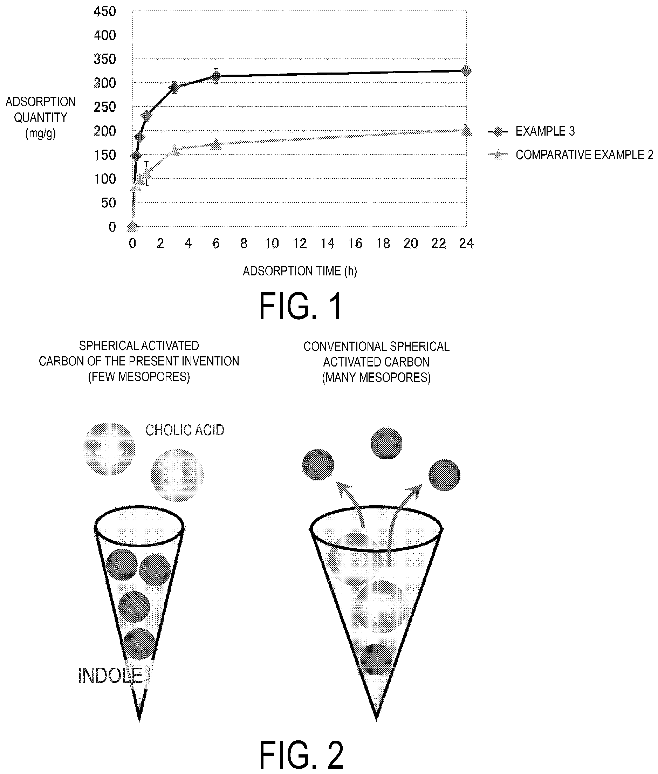

FIG. 2 is a schematic diagram of indole adsorption in the presence of cholic acid of the spherical activated carbon used in the present invention and of a conventional spherical activated carbon.

Because the molecular weight of indole is low, it is adsorbed by micropores of small pore diameter. On the other hand, since the molecular weight of cholic acid is high, it is adsorbed by mesopores of large pore diameter. It is thought that in a conventional spherical activated carbon, adsorption of cholic acid to mesopores inhibits adsorption of indole to micropores. On the other hand, since the spherical activated carbon of the present invention has few mesopores, the adsorption of cholic acid to mesopores is inhibited. For this reason, indole reaches the micropores unencumbered by cholic acid, and can be adsorbed by the micropores.

[2] Adsorbent for oral administration for treating or preventing a renal disease or a liver disease

Because the spherical activated carbon used for the adsorbent for oral administration of the present invention has excellent adsorbance of liver disease aggravating factors and toxic substances in renal diseases, the spherical activated carbon may be used as an adsorbent for oral administration for treating or preventing a renal disease or may be used as an adsorbent for oral administration for treating or preventing a liver disease.

Examples of the renal disease include chronic renal failure, acute renal failure, chronic pyelonephritis, acute pyelonephritis, chronic nephritis, acute nephritic syndrome, acute progressive nephritic syndrome, chronic nephritic syndrome, nephrotic syndrome, nephrosclerosis, interstitial nephritis, tubulopathy, lipoid nephrosis, diabetic nephropathy, renovascular hypertension, and hypertension syndrome, or secondary renal diseases attendant to these primary diseases. Another example is pre-dialysis mild renal failure, and it may be used in condition improvement of mild renal failure before dialysis or condition improvement during dialysis (see "Clinical Nephrology," Asakura Publishing, N. Honda, K. Koiso, K. Kurogawa, 1990 edition, and "Nephrology," Igaku Shoin, T. Onomae, S. Fujimi, editors, 1981 edition).

Examples of the liver disease include fulminant hepatitis, chronic hepatitis, viral hepatitis, alcoholic hepatitis, hepatic fibrosis, cirrhosis, hepatic cancer, autoimmune hepatitis, drug-induced allergic hepatitis, primary biliary cirrhosis, tremor, encephalopathy, metabolic disorder, and functional disorder. Otherwise, it may also be used in treatment of illnesses caused by toxic substances present in the body, that is, mental illness and the like.

Therefore, when the adsorbent for oral administration according to the present invention is used as a medicine for treating a renal disease, the adsorbent for oral administration contains the above spherical activated carbon as an active ingredient. When the adsorbent for oral administration of the present invention is used as a medicine for treating a renal disease or a medicine for treating a liver disease, the dosage thereof is influenced by whether the subject of administration is a human or other animal, and by age, individual differences, disease condition, or the like. Therefore, depending on the case, a dosage outside the following range may be appropriate, but in general, the orally administered dosage in humans is from 1 g to 20 g per day divided into three to four doses, and may be further adjusted according to symptoms. The administered form may be a powder, granules, tablet, sugar-coated pill, capsule, suspension, stick, individual package, emulsion, or the like. When ingested as a capsule, in addition to an ordinary gelatin capsule, an enteric-coated capsule may be used as necessary. When used as a tablet, it needs to be dissolved into microparticles in the body. Additionally, it may be used in the form of a complex blended with an electrolyte modifier such as alumigel or Kayexalate, which are other preparations.

[3] Method of treating a renal disease or liver disease

The spherical activated carbon used in the adsorbent for oral administration according to the present invention can be used in a method of treating or preventing a renal disease or a liver disease. Therefore, the method of treating a renal disease or a liver disease of the present invention is characterized in that the above adsorbent for oral administration containing the spherical activated carbon is administered in an effective dose to a renal disease or liver disease threatment subject.

The administration route, dosage, administration interval, and the like of the above spherical activated carbon may be determined as appropriate in accordance with the type of illness, the age, gender, and body weight of the patient, the degree of symptoms, the dosing method, and the like.

[4] A spherical activated carbon for use in method of treating of renal disease or liver disease

The spherical activated carbon used in the adsorbent for oral administration according to the present invention can be used in a method of treating or preventing a renal disease or a liver disease. Therefore, the spherical activated carbon of the present invention is for use in a method of treating or preventing a renal disease or a liver disease.

The amount and the like of the above spherical activated carbon used in prevention or treatment may be determined as appropriate in accordance with the type of illness, the age, gender, and body weight of the patient, the degree of symptoms, the dosing method, and the like.

[5] Use of a spherical activated carbon for production of a medicine for treating renal disease or liver disease

The spherical activated carbon used in the adsorbent for oral administration according to the present invention can be used for producing a medicine for treating or preventing a renal disease or a liver disease. Therefore, use of the present invention is use of the spherical activated carbon for producing a medicine for treating or preventing a renal disease or a liver disease.

The contained amount and the like of the above spherical activated carbon in the medicine for treatment or prevention may be determined as appropriate in accordance with the type of illness, the age, gender, and body weight of the patient, the degree of symptoms, the dosing method, and the like.

[6] Use of a spherical activated carbon for treating renal disease or liver disease

The spherical activated carbon used in the adsorbent for oral administration according to the present invention can be used for treating a renal disease or a liver disease. Therefore, use of the present invention is use of the spherical activated carbon for treating or preventing a renal disease or a liver disease.

The amount and the like of the above spherical activated carbon used in treatment or prevention may be determined as appropriate in accordance with the type of illness, the age, gender, and body weight of the patient, the degree of symptoms, the dosing method, and the like.

Examples

The present invention will be described in detail hereafter using examples, but these examples do not limit the scope of the present invention.

The physical property values, namely average particle diameter, bulk density, specific surface area, volume of pores, particle diameter distribution, total acidic group content, total basic group content, and indole adsorption test, of the spherical activated carbon used for the adsorbent for oral administration according to the present invention were measured by the following methods.

(1) Average particle diameter (Dv50)

The particle diameter at a cumulative particle diameter percentage of 50% on a volume standard cumulative particle diameter distribution curve created using a laser diffraction-style particle size distribution analyzer (SALAD-3000S; Shimadzu Corp.) was used as the average particle diameter (Dv50).

(2) Bulk density

Measurement was performed in accordance with the packing density measurement method of JIS K 1474-5.7.2.

(3) Specific surface area (specific surface area calculation method by BET method)

The gas adsorption quantity of a spherical activated carbon sample can be measured using a specific surface area analyzer that uses the gas adsorption method (for example, ASAP2010 or ASAP2020; Micromeritics Corp.), and a specific surface area can be calculated using the formula below. Specifically, a sample tube is packed with the spherical activated carbon sample, and after vacuum-drying at 350°C, post-drying sample weight is measured. Then, the sample tube is cooled to -196°C and nitrogen is introduced into the sample tube to adsorb nitrogen on the spherical activated carbon sample, and the relationship between nitrogen partial pressure and adsorbed quantity (adsorption isotherm) is measured.

A BET plot is created, with the relative pressure of nitrogen taken as p and the adsorbed quantity at that time taken as v ($cm^3$/g STP). Specifically, the range of p from 0.05 to 0.20 is plotted with p/(v(1-p)) on the vertical axis and p on the horizontal axis, and the specific surface area S (units: $m^2$/g) is determined by the following formula from the slope b (units: $g/cm^3$) and intercept c (units: $g/cm^3$) at that time.

[Equation 2]

$$S = \frac{MA \times (6.02 \times 10^{23})}{22414 \times 10^{18} \times (b+c)}$$

Here, MA was 0.162 $nm^2$ by nitrogen atom cross-sectional area.

(4) Volume of micropores by gas adsorption method (Saito-Foley calculation formula)

The relationship between nitrogen partial pressure and adsorbed quantity (adsorption isotherm) of a spherical activated carbon sample was measured at liquid nitrogen temperature (-196°C) using a specific surface area analyzer that uses the gas adsorption method (ASAP2010 or ASAP2020; Micromeritics Corp.). From the obtained adsorption isotherm, pore distribution was calculated by the Saito-Foley calculation formula (Saito, A. and Foley, H.C., AIChE Journal 37 (3), 429 (1991)) using analysis software that comes with the aforementioned specific surface area analyzer (ASAP2010 or ASAP2020; Micromeritics Corp.). Pore shape analyzed by slit geometry was that obtained by the original Horvath-Kawazoe calculation method (Horvath, G. and Kawazoe, K., J. Chem. Eng. Japan 16 (6), 470 (1983)), but since the structure of the carbon is a three-dimensionally disarrayed structure of non-graphitizable carbon, calculation by cylinder geometry (Saito, A. and Foley, H.C., AIChE Journal 37 (3), 429 (1991)) was chosen.

The various parameters used in the calculation are as follows.

Interaction parameter: 1.56 $\times$ $10^{-43}$ ergs·$cm^4$
Diameter of adsorptive molecule: 0.3000 nm
Diameter of sample molecule: 0.3400 nm
Density conversion factor: 0.001547 ($cm^3$ liquid/$cm^3$ STP)

(5) Volume of mesopores by mercury penetration method

Volume of pores can be measured using a mercury porosimeter (for example, Autopore 9200; Micromeritics Corp.). The spherical activated carbon sample is put in a sample container, and degassed for 30 minutes under pressure not higher than 2.67 Pa. Then, mercury is introduced into the sample container, pressure is gradually increased, and the mercury penetrates into the pores of the spherical activated carbon sample (maximum pressure: 414 MPa). From the relationship between pressure and mercury penetration quantity at this time, the pore volume distribution of the spherical activated carbon sample is measured using the calculation formulas below.

Specifically, the volume of mercury that penetrates the spherical activated carbon sample is measured from a pressure equivalent to pore diameter 21 $\mu$m (0.06 MPa) to the maximum pressure (414 MPa, equivalent to pore diameter 3 nm). In the calculation of pore diameter, when mercury penetrates into the pores of a cylinder having a diameter (D) at a pressure (P), a surface tension of mercury is balanced with a pressure acting on a cross section of the pores and the following equation holds true:

$$-\pi D \gamma \cos\theta = \pi (D/2)^2 \cdot P,$$

where the surface tension of mercury is taken as "$\gamma$" and the contact angle between mercury and the pore wall is taken as "$\theta$".

Therefore,

$$D = (-4\gamma\cos\theta)/P.$$

In the present specification, the surface tension of mercury is taken as 484 dyne/cm and the contact angle between mercury and carbon is taken as 130 degrees. When the pressure P is expressed in MPa and the pore diameter D is expressed in $\mu$m, the relationship between the pressure P and the pore diameter D is determined by using the following formula:

$$D = 1.24/P.$$

For example, the volume of pores having a diameter in the range of from 20 nm to 10000 nm is equivalent to the volume of mercury that penetrates at mercury penetration pressure from 0.124 MPa to 62 MPa. The volume of pores having a diameter in the range of from 7.5 nm to 15000 nm is equivalent to the volume of mercury that penetrates at mercury penetration pressure from 0.083 MPa to 165 MPa. The volume of pores having a diameter in the range of from 3 nm to 20 nm is equivalent to the volume of mercury that penetrates at mercury penetration pressure from 413 MPa to 62 MPa.

(6) Particle diameter distribution

The number particle diameter distribution was measured using a laser diffraction-type particle size distribution analyzer (SALAD-3000S; Shimadzu Corp.). The typical particle diameter D of measured particle diameter classification and the number of particles n in that measured particle diameter classification were determined, and the length average particle diameter Di and the weight average particle diameter $D_4$ were calculated by the following formulae.

[Equation 3]

$$D_1 = \frac{\sum(nD)}{\sum n}$$

[Equation 4]

$$D_4 = \frac{\sum(nD^4)}{\sum(nD^3)}$$

(7) Indole adsorption test

An indole adsorption test and an indole adsorption test in the presence of cholic acid were performed by the following methods on the spherical activated carbons obtained in the examples and comparative examples.

One gram of a dried spherical activated carbon was accurately added to a container containing 900 mL of a degassed indole solution having an indole concentration of 500 mg/L prepared as the second liquid of an elution test or of a solution having an indole concentration of 500 mg/L and a sodium cholate concentration of 15 mmol/L. Using an elution test container, it was tested for 24 hours at 37°C at a paddle rotation rate of 50 rpm. 24 hours after the start of the test, 10 mL was sampled, the spherical activated carbon was filtered using a membrane filter, and the residual concentration of indole in the filtrate was measured by liquid chromatography. The residual concentration of indole was determined from a separately produced calibration curve, and based on this, the adsorbed quantity (mg/g) of indole per gram of the spherical activated carbon was calculated from the following formula.

$$\text{Adsorbed quantity (mg/g) of indole per gram of the spherical activated carbon} = (500 \text{ (mg/L)} - \text{residual concentration (mg/L)}) \times 0.9 \text{ (L)/quantity of the activated carbon (g)}$$

Example 1

4338 g of ion-exchanged water, 6 g of sodium nitrite, and 169 g of 4 wt% aqueous solution of Metalose 60SH-15 (manufactured by Shin-Etsu Chemical Co., Ltd.) were put in a 10-L polymerization reactor. To this were added 582 g of styrene, 393 g of divinylbenzene (57% divinylbenzene and 43% ethylvinylbenzene), 525 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring to disperse and suspend, and then held in that state for 20 hours. The obtained resin was washed with water and filtered, and then dried for 16 hours at 200°C under nitrogen flow, to produce spherical porous synthetic resin having an average particle diameter of 195 μm.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 290°C in 9 hours, and spherical porous oxidized resin was thereby obtained. This was heat treated at 850°C in a nitrogen atmosphere, and a spherical carbon of bulk density 0.83 g/mL was obtained. Using a fluidized bed, activation treatment was performed on the obtained spherical carbon at 900°C in a nitrogen atmosphere containing steam until the BET specific surface area reached 1850 m$^2$/g, and a spherical activated carbon was thereby obtained. The characteristics of the obtained spherical activated carbon are shown in Table 1.

Example 2

4338 g of ion-exchanged water, 6 g of sodium nitrite, and 169 g of 4 wt% aqueous solution of Metalose 60SH-15 (manufactured by Shin-Etsu Chemical Co., Ltd.) were put in a 10-L polymerization reactor. To this were added 582 g of styrene, 393 g of divinylbenzene (57% divinylbenzene and 43% ethylvinylbenzene), 525 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring to disperse and suspend, and then held in that state for 20 hours. The obtained resin was washed with water and filtered, and then dried for 16 hours at 200°C under nitrogen flow, to produce spherical porous synthetic resin having an average particle diameter of 245 μm.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 290°C in 9 hours, and spherical porous oxidized resin was thereby obtained. This was heat treated at 850°C in a nitrogen atmosphere, and a spherical carbon of bulk density 0.83 g/mL was obtained. Using a fluidized bed, activation treatment was performed on the obtained spherical carbon at 900°C in a nitrogen atmosphere containing steam until the BET specific surface area reached 1790 m$^2$/g, and a spherical activated carbon was thereby obtained. The characteristics of the obtained spherical activated carbon are shown in Table 1.

Example 3

4338 g of ion-exchanged water, 6 g of sodium nitrite, and 169 g of 4 wt% aqueous solution of Metalose 60SH-15 (manufactured by Shin-Etsu Chemical Co., Ltd.) were put in a 10-L polymerization reactor. To this were added 582 g of styrene, 393 g of divinylbenzene (57% divinylbenzene and 43% ethylvinylbenzene), 525 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring to disperse and suspend, and then held in that state for 20 hours. The obtained resin was washed with water and filtered, and then dried for 16 hours at 200°C under nitrogen flow, to produce a spherical porous synthetic resin having an average particle diameter of 197 $\mu$m.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 290°C in 9 hours, and spherical porous oxidized resin was thereby obtained. This was heat treated at 850°C in a nitrogen atmosphere, and a spherical carbon of bulk density 0.83 g/mL was obtained. Using a fluidized bed, activation treatment was performed on the obtained spherical carbon at 900°C in a nitrogen atmosphere containing steam until the BET specific surface area reached 1670 $m^2$/g, and a spherical activated carbon was thereby obtained. The characteristics of the obtained spherical activated carbon are shown in Table 1.

Example 4

4338 g of ion-exchanged water, 6 g of sodium nitrite, and 169 g of 4 wt% aqueous solution of Metalose 60SH-15 (manufactured by Shin-Etsu Chemical Co., Ltd.) were put in a 10-L polymerization reactor. To this were added 582 g of styrene, 393 g of divinylbenzene (57% divinylbenzene and 43% ethylvinylbenzene), 525 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring to disperse and suspend, and then held in that state for 20 hours. The obtained resin was washed with water and filtered, and then dried for 16 hours at 200°C under nitrogen flow, to produce spherical porous synthetic resin having an average particle diameter of 200 $\mu$m.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 290°C in 9 hours, and spherical porous oxidized resin was thereby obtained. This was heat treated at 850°C in a nitrogen atmosphere, and a spherical carbon of bulk density 0.83 g/mL was obtained. Using a fluidized bed, activation treatment was performed on the obtained spherical carbon at 900°C in a nitrogen atmosphere containing steam until the BET specific surface area reached 1280 $m^2$/g, and a spherical activated carbon was thereby obtained. The characteristics of the obtained spherical activated carbon are shown in Table 1.

Example 5

4338 g of ion-exchanged water, 6 g of sodium nitrite, and 169 g of 4 wt% aqueous solution of Metalose 60SH-15 (manufactured by Shin-Etsu Chemical Co., Ltd.) were put in a 10-L polymerization reactor. To this were added 582 g of styrene, 393 g of divinylbenzene (57% divinylbenzene and 43% ethylvinylbenzene), 525 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring to disperse and suspend, and then held in that state for 20 hours. The obtained resin was washed with water and filtered, and then dried for 16 hours at 200°C under nitrogen flow, to produce spherical porous synthetic resin having an average particle diameter of 165 $\mu$m.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 290°C in 9 hours, and spherical porous oxidized resin was thereby obtained. This was heat treated at 850°C in a nitrogen atmosphere, and a spherical carbon of bulk density 0.83 g/mL was obtained. Using a fluidized bed, activation treatment was performed on the obtained spherical carbon at 900°C in a nitrogen atmosphere containing steam until the BET specific surface area reached 850 $m^2$/g, and a spherical activated carbon was thereby obtained. The characteristics of the obtained spherical activated carbon are shown in Table 1.

Example 6

4338 g of ion-exchanged water, 6 g of sodium nitrite, and 169 g of 4 wt% aqueous solution of Metalose 60SH-15 (manufactured by Shin-Etsu Chemical Co., Ltd.) were put in a 10-L polymerization reactor. To this were added 582 g of styrene, 393 g of divinylbenzene (57% divinylbenzene and 43% ethylvinylbenzene), 525 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring to disperse and suspend, and then held in that state for 20 hours. The obtained resin was washed with water and filtered, and then dried for 16 hours at 200°C under nitrogen flow, to produce spherical porous synthetic resin having an average particle diameter of 250 $\mu$m.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 290°C in 9 hours, and spherical porous oxidized resin was thereby obtained. This was heat treated at 850°C in a nitrogen atmosphere, and a spherical carbon of bulk density 0.83 g/mL was obtained. Using a fluidized bed, activation treatment was performed on the obtained spherical carbon at 900°C in a nitrogen atmosphere containing steam until the BET specific surface area reached 900 $m^2$/g, and a spherical activated carbon was thereby obtained. The characteristics of the obtained spherical activated carbon are shown in Table 1.

Comparative Example 1

4567 g of ion-exchanged water and 249 g of methylcellulose were put in a 10-L polymerization vessel. To this were added 481 g of styrene, 1119 g of 57% pure divinylbenzene (57% divinylbenzene and 43% ethylvinylbenzene), 9.3 g of 2,2'-azobis(2,4-dimethylvaleronitrile), and 560 g of hexane as a porogen, and the interior of the system was then replaced with nitrogen gas.

This two-phase system was heated to 55°C while stirring to disperse and suspend, and then held in that state for 20 hours. The obtained resin was washed with water and filtered, and then dried for 16 hours at 200°C under nitrogen flow, to produce spherical porous synthetic resin having an average particle diameter of 157 $\mu$m.

The obtained spherical porous synthetic resin was put in a reactor with a grating, and infusibility treatment was performed in a vertical tube furnace. As the infusibility treatment, dry air was made to flow from bottom to top of the reaction tube, and after heating to 180°C, the temperature was raised from 180°C to 240°C in 3 hours, and then held at 240°C for 1 hour. The temperature was then raised from 240°C to 260°C in 1 hour and held at 260°C for 5 hours, and then raised from 260°C to 300°C in 2 hours and held at 300°C for 1 hour, and spherical porous oxidized resin was thereby obtained. This spherical porous oxidized resin was heated in a nitrogen atmosphere at 850°C, and then, using a fluidized bed, activation treatment was performed in a nitrogen atmosphere containing steam until the BET specific surface area reached 2660 $m^2$/g, and a spherical activated carbon was thereby obtained.

Comparative Example 2

4338 g of ion-exchanged water, 6 g of sodium nitrite, and 169 g of 4 wt% aqueous solution of Metalose 60SH-15 (manufactured by Shin-Etsu Chemical Co., Ltd.) were put in a 10-L polymerization reactor. To this were added 432 g of styrene, 393 g of divinylbenzene (57% divinylbenzene and 43% ethylvinylbenzene), 675 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring to disperse and suspend, and then held in that state for 20 hours. The obtained resin was washed with water and filtered, and then dried for 16 hours at 200°C under nitrogen flow, to produce spherical porous synthetic resin having an average particle diameter of 189 $\mu$m.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 240°C in 3 hours, and then held at 240°C for 1 hour. The temperature was then raised from 240°C to 260°C in 1 hour and held at 260°C for 5 hours, and then raised from 260°C to 300°C in 2 hours and held at 300°C for 1 hour, and spherical porous oxidized resin was thereby obtained. This was heat treated at 850°C in a nitrogen atmosphere, and a spherical carbon of bulk density 0.75 g/mL was obtained. Using a fluidized bed, activation treatment was performed on the obtained spherical carbon at 850°C in a nitrogen atmosphere containing steam until the BET specific surface area reached 1650 $m^2$/g, and a spherical activated carbon was thereby obtained. The characteristics of the obtained spherical activated carbon are shown in Table 1.

Comparative Example 3

4338 g of ion-exchanged water, 6 g of sodium nitrite, and 169 g of 4 wt% aqueous solution of Metalose 60SH-15 (manufactured by Shin-Etsu Chemical Co., Ltd.) were put in a 10-L polymerization reactor. To this were added 582 g of styrene, 393 g of divinylbenzene (57% divinylbenzene and 43% ethylvinylbenzene), 525 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring to disperse and suspend, and then held in that state for 20 hours. The obtained resin was washed with water and filtered, and then dried for 16 hours at 200°C under nitrogen flow, to produce spherical porous synthetic resin having an average particle diameter of 170 $\mu$m.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 290°C in 9 hours, and spherical porous oxidized resin was thereby obtained. This was heat treated at 850°C in a nitrogen atmosphere, and a spherical carbon of bulk density 0.83 g/mL was obtained. Using a fluidized bed, activation treatment was performed on the obtained spherical carbon at 850°C in a nitrogen atmosphere containing steam until the BET specific surface area reached 2050 $m^2$/g, and a spherical activated carbon was thereby obtained. The characteristics of the obtained spherical activated carbon are shown in Table 1.

Comparative Example 4

4338 g of ion-exchanged water, 6 g of sodium nitrite, and 169 g of 4 wt% aqueous solution of Metalose 60SH-15 (manufactured by Shin-Etsu Chemical Co., Ltd.) were put in a 10-L polymerization reactor. To this were added 582 g of styrene, 393 g of divinylbenzene (57% divinylbenzene and 43% ethylvinylbenzene), 525 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring to disperse and suspend, and then held in that state for 20 hours. The obtained resin was washed with water and filtered, and then dried for 16 hours at 200°C under nitrogen flow, to produce spherical porous synthetic resin having an average particle diameter of 164 μm.
The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 290°C in 9 hours, and spherical porous oxidized resin was thereby obtained. This was heat treated at 850°C in a nitrogen atmosphere, and a spherical carbon of bulk density 0.83 g/mL was obtained. Using a fluidized bed, activation treatment was performed on the obtained spherical carbon at 900°C in a nitrogen atmosphere containing steam until the BET specific surface area reached 540 $m^2$/g, and a spherical activated carbon was thereby obtained. The characteristics of the obtained spherical activated carbon are shown in Table 1.

Comparative Example 5

4338 g of ion-exchanged water, 6 g of sodium nitrite, and 169 g of 4 wt% aqueous solution of Metalose 60SH-15 (manufactured by Shin-Etsu Chemical Co., Ltd.) were put in a 10-L polymerization reactor. To this were added 582 g of styrene, 393 g of divinylbenzene (57% divinylbenzene and 43% ethylvinylbenzene), 525 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring to disperse and suspend, and then held in that state for 20 hours. The obtained resin was washed with water and filtered, and then dried for 16 hours at 200°C under nitrogen flow, to produce spherical porous synthetic resin having an average particle diameter of 169 μm.
The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 290°C in 9 hours, and spherical porous oxidized resin was thereby obtained. This was heat treated at 850°C in a nitrogen atmosphere, and a spherical carbon of bulk density 0.83 g/mL was obtained. Using a fluidized bed, activation treatment was performed on the obtained spherical carbon at 900°C in a nitrogen atmosphere containing steam until the BET specific surface area reached 340 $m^2$/g, and a spherical activated carbon was thereby obtained. The characteristics of the obtained spherical activated carbon are shown in Table 1.

Table 1

| | Bulk density | BET specific surface area | Average particle diameter | Volume of pores having a diameter less than 3 nm | Volume of pores having a diameter from 3 nm to 50 nm | Micropore/ mesopore ratio | Indole adsorption quantity in presence of cholic acid (24 h) | Indole adsorption quantity in absence of cholic acid (24 h) |
|---|---|---|---|---|---|---|---|---|
| Example 1 | 0.461 | 1850 | 98 | 0.84 | 0.28 | 3.00 | 310 | 395 |
| Example 2 | 0.482 | 1790 | 130 | 0.84 | 0.25 | 3.36 | 344 | 390 |
| Example 3 | 0.509 | 1670 | 102 | 0.76 | 0.20 | 3.80 | 349 | 379 |
| Example 4 | 0.585 | 1280 | 107 | 0.60 | 0.09 | 6.67 | 360 | 369 |
| Example 5 | 0.671 | 850 | 92 | 0.39 | 0.03 | 13.0 | 320 | 335 |
| Example 6 | 0.700 | 920 | 144 | 0.42 | 0.04 | 10.5 | 338 | 343 |
| Comparative Example 1 | 0.300 | 2660 | 77 | 1.45 | 1.04 | 1.39 | 221 | 396 |
| Comparative Example 2 | 0.439 | 1650 | 104 | 0.74 | 0.37 | 1.89 | 202 | 380 |
| Comparative Example 3 | 0.426 | 2050 | 93 | 0.93 | 0.40 | 2.33 | 275 | 392 |
| Comparative Example 4 | 0.770 | 540 | 94 | 0.25 | 0.01 | 25.0 | 140 | 152 |
| Comparative Example 5 | 0.820 | 340 | 100 | 0.16 | 0.01 | 16.0 | 30 | 51 |

As shown in Table 1, the spherical activated carbon of Examples 1 to 6, which had a BET specific surface area of 800 m$^2$ or more, a bulk density of from 0.3 g/mL to 0.8 g/mL, a volume of pores having a diameter less than 3 nm of 0.3 mL/g or more, and a micropore/mesopore ratio of 3.0 or more, exhibited excellent indole adsorption ability in the presence of cholic acid. However, the spherical activated carbon of Comparative Examples 1 to 5, which did not have the above-mentioned physical properties, exhibited a certain indole adsorption ability in the absence of cholic acid, but indole adsorption ability in the presence of cholic acid was markedly lower.

Industrial Applicability

The adsorbent for oral administration of the present invention may be used as an adsorbent for oral administration for treating or preventing a renal disease or may be used as an adsorbent for treating or preventing a liver disease. Examples of the renal disease include chronic renal failure, acute renal failure, chronic pyelonephritis, acute pyelonephritis, chronic nephritis, acute nephritic syndrome, acute progressive nephritic syndrome, chronic nephritic syndrome, nephrotic syndrome, nephrosclerosis, interstitial nephritis, tubulopathy, lipoid nephrosis, diabetic nephropathy, renovascular hypertension, and hypertension syndrome, or secondary renal diseases attendant to these primary diseases. Another example is pre-dialysis mild renal failure, and it may be used in condition improvement of mild renal failure before dialysis or condition improvement during dialysis (see "Clinical Nephrology," Asakura Publishing, N. Honda, K. Koiso, K. Kurogawa, 1990 edition, and "Nephrology," Igaku Shoin, T. Onomae, S. Fujimi, editors, 1981 edition). Examples of the liver disease include fulminant hepatitis, chronic hepatitis, viral hepatitis, alcoholic hepatitis, hepatic fibrosis, cirrhosis, hepatic cancer, autoimmune hepatitis, drug-induced allergic hepatitis, primary biliary cirrhosis, tremor, encephalopathy, metabolic disorder, and functional disorder. Otherwise, it may also be used in treatment of illnesses caused by toxic substances present in the body, that is, mental illness and the like.
The present invention has been described above using specific modes of embodiment, but modifications and improvements apparent to persons having ordinary skill in the art are also included in the scope of the present invention.

**Claims**

1. An adsorbent for oral administration comprising a spherical activated carbon, wherein the activated carbon has a specific surface area determined by the BET method of 800 m$^2$/g or more, a bulk density of from 0.3 g/mL to 0.8 g/mL, a volume of pores having a diameter less than 3 nm of 0.3 mL/g or more, and a micropore/mesopore ratio (Vm) determined by Formula (1):

$$Vm = Vmic/Vmet \ (1)$$

wherein Vmic is a volume of pores having a diameter less than 3 nm, and Vmet is a volume of pores having a diameter from 3 nm to 50 nm;
of 3.0 or more.

2. The adsorbent for oral administration according to claim 1, wherein an average particle diameter of the spherical activated carbon is from 50 $\mu$m to 200 $\mu$m.

3. The adsorbent for oral administration according to claim 1 or 2, wherein the spherical activated carbon is prepared from a crosslinked vinyl resin as a carbon source.

4. An agent for treating or preventing a renal disease comprising, the adsorbent for oral administration according to any one of claims 1 to 3 as an active ingredient.

5. An agent for treating or preventing a liver disease comprising, the adsorbent for oral administration according to any one of claims 1 to 3 as an active ingredient.

FIG. 1

FIG. 2

## INTERNATIONAL SEARCH REPORT

International application No.

PCT/JP2015/074213

| A. CLASSIFICATION OF SUBJECT MATTER |
|---|
| $A61K33/44$ (2006.01)i, $A61P1/16$ (2006.01)i, $A61P13/12$ (2006.01)i, $A61P39/02$ (2006.01)i |

According to International Patent Classification (IPC) or to both national classification and IPC

| B. FIELDS SEARCHED |
|---|
| Minimum documentation searched (classification system followed by classification symbols) |
| A61K33/44, A61P1/16, A61P13/12, A61P39/02 |

Documentation searched other than minimum documentation to the extent that such documents are included in the fields searched

Jitsuyo Shinan Koho     1922-1996    Jitsuyo Shinan Toroku Koho   1996-2015
Kokai Jitsuyo Shinan Koho   1971-2015    Toroku Jitsuyo Shinan Koho   1994-2015

Electronic data base consulted during the international search (name of data base and, where practicable, search terms used)

CAplus/REGISTRY/MEDLINE/EMBASE/BIOSIS(STN),
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| Y | JP 2005-314415 A (Kureha Chemical Industry Co., Ltd.), 10 November 2005 (10.11.2005), claims; paragraphs [0001] to [0008], [0020] to [0021], [0046] to [0047]; examples; table 1 & US 2008/0044477 A1 & WO 2005/094844 A1 & EP 1745792 A1 & KR 10-2006-0135011 A & CN 1938037 A & RU 2006138608 A | 1-5 |
| Y | JP 2006-131461 A (Organo Corp.), 25 May 2006 (25.05.2006), claims; paragraphs [0010] to [0011], [0020], [0042], [0044], [0051]; examples; tables 1 to 2 (Family: none) | 1-5 |

| ☒ Further documents are listed in the continuation of Box C. | ☐ See patent family annex. |
|---|---|

| * Special categories of cited documents: |  |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| 28 September 2015 (28.09.15) | 06 October 2015 (06.10.15) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (July 2009)

**INTERNATIONAL SEARCH REPORT**

International application No.

PCT/JP2015/074213

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2010/086985 A1 (Asahi Organic Chemicals Industry Co., Ltd.), 05 August 2010 (05.08.2010), claims; paragraphs [0002] to [0010], [0019] to [0020]; examples; tables 1 to 2 (Family: none) | 1-5 |
| A | WO 2013/051680 A1 (Teijin Pharma Ltd.), 11 April 2013 (11.04.2013), claims; paragraphs [0001] to [0010], [0023] to [0025]; examples; table 1 & US 2014/0242147 A1 & EP 2772261 A1 & CN 103841981 A & KR 10-2014-0072077 A | 1-5 |
| P,A | WO 2014/129614 A1 (Kureha Corp.), 28 August 2014 (28.08.2014), claims; paragraphs [0001] to [0008], [0019]; examples; tables 1 to 2 & TW 201440819 A | 1-5 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP S6211611 B
- JP 2006273772 A
- JP 2007045775 A

### Non-patent literature cited in the description

- *Japanese Journal of Nephrology,* 1990, vol. XXXII (6), 65-71
- *Japanese Journal of Clinical Dialysis,* 1998, vol. 14 (4), 433-438
- **N. HONDA ; K. KOISO ; K. KUROGAWA.** Clinical Nephrology. Asakura Publishing, 1990
- Nephrology. Igaku Shoin, 1981
- **SAITO, A. ; FOLEY, H.C.** *AIChE Journal,* 1991, vol. 37 (3), 429
- **HORVATH, G. ; KAWAZOE, K.** *J. Chem. Eng.,* 1983, vol. 16 (6), 470
- **IGAKU SHOIN ; T. ONOMAE ; S. FUJIMI.** Nephrology. 1981